# EUROPÄISCHE PATENTANMELDUNG

(11) **EP 0 955 360 A2**
(43) Veröffentlichungstag der Anmeldung: **10.11.1999**
(21) Anmeldenummer: 99105183.0
(22) Anmeldetag: 31.03.1999
(51) Int. Cl.: C12N 5/10, C12N 5/12, C12N 5/06, C12N 5/08

(54) **Verfahren zur Immortalisation von Zellen mit Hilfe konditional transformierter Helferzellen**

(30) Priorität: 09.04.1998 DE 19816116
(71) Anmelder: GSF-Forschungszentrum für Umwelt und Gesundheit, GmbH, 85764 Oberschleissheim (DE)
(72) Erfinder: Staege, Martin, 80689 München (DE); Bornkamm, Georg, 81243 München (DE); Kempes, Bettina, 80687 München (DE)
(74) Vertreter: Reinhard - Skuhra - Weise & Partner

(57) **Zusammenfassung**

Die Erfindung betrifft ein Verfahren zur Herstellung einer konditional immortalisierten Immortalisationshelferzelle (Fusem), die durch dieses Verfahren hergestellte Fuseme, mit Hilfe der Fuseme hergestellte Hybridomzellen sowie ein Verfahren zur Immortalisation von Säugerzellen mit Hilfe der Fusemzellen. Die Erfindung betrifft weiterhin ein Verfahren zur Generierung von T-Zellen, die gegen Tumorzellen gerichtet sind unter Verwendung einer Fusemzelle.

## Beschreibung

Die Erfindung betrifft ein Verfahren zur Herstellung einer konditional immortalisierten Immortalisations-Helferzelle (Fusem), die durch dieses Verfahren hergestellten Fuseme, mit Hilfe der Fuseme hergestellte Hybridomzellen sowie ein Verfahren zur Immortalisation von Säugerzellen mit Hilfe der Fusemzellen. Die Erfindung betrifft weiterhin ein Verfahren zur Generierung von T-Zellen, die gegen Tumorzellen gerichtet sind unter Verwendung einer Fusemzelle.

Bei zahlreichen medizinischen und biologischen Fragestellungen besteht die Notwendigkeit oder der Wunsch, ausgehend von wenigen Zellen eine große Zahl dieser Zellen *in vitro* zu erzeugen, um mit diesen wissenschaftliche Experimente durchführen zu können oder im Rahmen therapeutischer Interventionen mit diesen Zellen zu arbeiten. Diese Expansion von Zellen stellt oft ein Problem dar, da normalerweise die Wachstumskapazität von Zellen beschränkt ist. Dieses gilt beispielsweise auch für zahlreiche Tumorzellen, welche oftmals *in vitro* einen Wachstumsnachteil gegenüber normalen Zellen haben, so daß eine Expansion dieser Zellen schwierig ist [Lange (1998), Visonneau et al. (1995)]. Eine Möglichkeit der Expansion von Zellen *in vitro* besteht darin, die Zellen mit geeigneten Stimuli (Zytokinen, geeigneten Feeder-Zellen, Stimulation von geeigneten Rezeptoren auf der Zelloberfläche, etc.) zu reizen, so daß das Wachstum bzw. Überleben dieser Zellen ermöglicht wird. Jedoch gilt auch hier, daß die Expansion im allgemeinen nicht beliebig möglich ist und die Zellen nach einiger Zeit das Wachstum einstellen. Darüber hinaus sind die Stimulationsbedingungen für viele Zelltypen noch nicht bekannt, so daß dieser Weg nicht universell einsetzbar ist. Zwar ist die "Kultur" verschiedener Tumorzellen in lebenden, immundefizienten Versuchstieren für längere Zeit möglich [Visonneau et al. (1995)], dieser Weg ist jedoch umständlich und darüberhinaus aus ethischen Gründen grundsätzlich abzulehnen.

Ein eleganter Weg dieses Problem zu umgehen ist die genetische Immortalisation der gewünschten Zellen [Hubbard & Ozer (1995), MacDonald (1994)]. Manche Tumorzell-Linien besitzen die Eigenschaft, auch *in-vitro* ein praktisch unbegrenztes Teilungsvermögen zu besitzen, also immortalisiert zu sein. Dieses führte in früherer Zeit zu der Entwicklung einer Technik der Zellfusion zwischen den Tumorzellen und den gewünschten zu immortalisierenden Zellen, welche in der bahnbrechenden Entwicklung der monoklonalen Antikörper mündete [Köhler & Milstein (1975), Peters et al. (1988)].

Insbesondere für die Immortalisation humaner Zellen stehen jedoch bis heute keine optimalen Fusionspartner zur Verfügung [Gordon (1989)]. Daher wird oft der Weg beschritten, humane Zellen mit geeigneten Zellen von Nagern zu fusionieren. Die entstehenden Hetero-Spezies-Hybridome haben jedoch die unangenehme Eigenschaft, zytogenetisch instabil zu sein und mit der Zeit einzelne (oft gerade die wichtigen und bei Mensch/Nager-Hybridomen bevorzugt die menschlichen) Chromosomen zu verlieren.

Eine Gruppe vielversprechender humaner Fusionsparter wird durch Epstein-Barr Virus (EBV)-transformierte lymphoblastoide Zell-Linien (LCL) repräsentiert. Insbesondere sind diese Zellen leicht etablierbar [Walls und Crawford (1987)]. Für zahlreiche Anwendungen haben diese Zellen jedoch unerwünschte Eigenschaften. So führt die Expression mancher viraler Proteine zu einer Inhibition der Immunglobulin-Synthese, eine Eigenschaft, die sich z. B. bei der Generierung humaner monoklonaler Antikörper hinderlich erweist. Daneben induzieren die Antigene, welche durch EBV in LCL exprimiert werden, eine starke Immunantwort, die alle übrigen Immunantworten überdecken kann. Dieses ist von Nachteil, wenn gewünscht ist, eine Fusion zwischen einer LCL und einer Tumorzelle durchzuführen, um anschließend das Hybridom zur Stimulation einer Immunantwort gegen Antigene der Tumorzelle einzusetzen. Eine andere Eigenschaft dieser Zellen erweist sich jedoch als besonders günstig, um eine Immunantwort zu induzieren. LCL gehören in ihren immunstimulatorischen Fähigkeiten zu den potentesten Zellen überhaupt. Es kann daher erwartet werden, daß Hybridome aus einer Tumorzelle und einer LCL neben den tumorspezifischen Antigenen auch über alle kostimulatorischen Moleküle verfügen, um eine Immunantwort effizient zu induzieren.

Es ist eine Aufgabe der vorliegenden Erfindung, ein Verfahren bereitzustellen, mit dessen Hilfe Zellen herstellbar sind, die dazu benutzt werden können, um die Information für die Immortalisation aufeine andere Zelle zu übertragen.

Diese Aufgabe wird erfindungsgemäß durch das im Anspruch 1 näher gekennzeichnete Verfahren gelöst, mit dessen Hilfe konditional immortalisierte Zellen bereitgestellt werden, die nachfolgend als "Fusem" bezeichnet werden. Diese Zelle kann als Helferzelle angesehen werden, da sie "hilft", eine andere Zelle zu immortalisieren. Das Verfahren zur Herstellung einer konditional immortalisierten Immortalisations-Helferzelle, hier als Fusem bezeichnet, weist zumindest die nachfolgenden Schritte auf:
(a) Einführen von Immortalisierungs-Genen in Säugerzellen in solcher Weise, daß zumindest die Expression und/oder Funktion zumindest eines dieser Gene regulierbar ist, um konditional immortalisierte Säugerzellen zu erhalten;
(b) Einführen von mindestens zwei Selektionsmarkern für eine Positiv- und eine Negativselektion, um eine Selektion zwischen den Fusemzellen, den mit Hilfe der Fusemzellen zu immortalisierenden Zellen und den mit Hilfe der Fusemzellen immortalisierten Zellen zu ermöglichen;
(c) Selektion auf solche Zellen (Fuseme), die die im Verfahrensschritt (b) eingeführten Selektionsmarker tragen und konditional immortalisiert sind.

Als Säugerzellen können beliebige Zellen eingesetzt werden, die immortalisierbar sind. Bevorzugt handelt es sich um menschliche Zellen oder Zellen aus Nagern. Beispiele für solche Zellen sind Lymphozytenzellen oder Fibroblastenzellen. Als Lymphozytenzellen können beispielsweise B-Zellen oder T-Zellen konditional immortalisiert werden.

Die konditionale Immortalisierung von Saugerzellen ist an sich bekannt [Kempkes et al. (1995), Wyllie et al. (1993)]. Hierzu müssen diejenigen Gene, die zur Immortalisierung einer Zelle notwendig sind, in eine Säugerzelle eingeführt werden. Das Einführen der Gene kann beispielsweise durch Infektion mit einem die Gene enthaltenden Virus oder durch Transfektion der DNA erfolgen.

Gene, die zur Immortalisierung von Zellen befähigt sind, sind an sich bekannt. Hierzu gehören beispielsweise die Immortalisierungsgene von EBV, von Adenoviren, HTLV-1 oder Onkogene. Die Gene können mit Hilfe von Vektoren in die konditional zu immortalisierenden Säugerzellen eingeführt werden, wobei erfindungsgemäß unter Vektoren Viren, Plasmide, Cosmide usw. fallen. Die für die Immortalisation verantwortlichen Gene werden jeweils so manipuliert, daß eine konditionale Expression oder Regulation der Funktion der Immortalisierungsgene ermöglicht wird. Beispielsweise können diese Gene auf einem Virus-Genom, auf dem sie natürlicherweise vorkommen, beispielsweise auf EBV, deletiert werden; die für die Immortalisierung notwendige Information wird dann beispielsweise in ein Plasmid eingeführt, und die Expression der Immortalisierungsgene auf dem Plasmid oder die Funktion dieser Gene kann dann durch geeignete Methoden, die in dieser Anmeldung beispielhaft dargestellt sind, konditional gesteuert werden. Beispiele für Vektoren zum Einführen von Immortalisierungsgenen sind EBV, Adenoviren, Retroviren, Foamyviren, Poxviren oder SV40 und Vektoren, die sich von diesen Viren ableiten. Ein Beispiel für einen von EBV-abgeleiteten Vektor sind mini-EBV-Vektoren. Ein Beispiel für einen Poxvirus ist das Vacciniavirus.

Die Immortalisierungsgene der oben genannten Viren bzw. die Onkogene, die eine Immortalisierung ermöglichen, sind an sich bekannte Beispiele für Onkogene, die zur Immortalisierung von Säugerzellen verwendbar sind, sind c-myc, c-abl, c-ras und Kombinationen dieser Onkogene; Immortalisierungsgene des EBV sind beispielsweise EBNA2 und LMP1.

Durch das erfindungsgemäße Verfahren wird eine Fusemzelle bereitgestellt, die zumindest durch die nachfolgenden Merkmale gekennzeichnet ist:
(a) Säugerzelle, die konditional immortalisiert ist;
(b) mit zumindest zwei Selektionsmarkern für eine Positiv- und eine Negativselektion, die eine Selektion zwischen Fusemzellen, zu immortalisierenden Zellen und immortalisierten Zellen ermöglichen.

Die durch die vorstehende Erfindung erhaltenenen Fusemzellen werden als Helferzellen eingesetzt, um Säugerzellen zu immortalisieren. Hierzu werden eine Fusemzelle und eine Säugerzelle, die mit Hilfe der Fusemzelle immortalisiert werden soll, derart zusammengebracht, daß das Merkmal der Immortalisation von den Fusemzellen auf die mit Hilfe der Fusemzellen zu immortalisierenden Zellen übertragen wird. Anschließend wird auf diejenigen Zellen selektiert, die das Merkmal der Immortalisation tragen. Dies kann beispielsweise dadurch erfolgen, daß das Merkmal der Immortalisation mit einem Selektionsmarker gekoppelt ist, auf den man selektioniert.

In einer anderen Ausführungsform sind die Fusemzellen immortalisiert und werden mit der zu immortalisierenden Zelle fusioniert, wodurch die Hybridzelle bereits in immortalisierter Form vorliegt. Außerdem kann eine Selektion auf wachsende Zellen erfolgen, da nur immortalisierte Zellen unbegrenzt wachsen können.

Als Säugerzelle, die immortalisiert werden soll, kann jede beliebige, immortalisierbare Säugerzelle eingesetzt werden. Beispiele hierfür sind Lymphozyten-Zellen oder Fibroblasten-Zellen. Beispiele für Lymphozyten-Zellen sind B-Zellen und T-Zellen. Als zu immortalisierende Zelle kann auch eine Tumorzelle benutzt werden. Ein Beispiel für eine Tumorzelle ist eine Leukämiezelle. In vitro haben Tumorzellen meist kein unbegrenztes Wachstumsvermögen. Dies liegt wohl meistens an unzureichenden Kulturbedingungen. So gelang beispielsweise beim malignen Melanom, einem Tumor, der als gut kultivierbar gilt, nur in ca. 25% der Fälle die Etablierung einer Zellinie aus dem Tumor.

Für die Übertragung des Merkmals der Immortalisation von den Fusemzellen auf die zu immortalisierenden Zelten werden zwei verschiedene Verfahren nachfolgend näher beschrieben. Beim ersten Verfahren werden von der Fusemzelle Virenpartikel freigesetzt, die die zur Immortalisation einer Zelle notwendigen Gene enthalten. Die Viruspartikel infizieren die zu immortalisierenden Zellen, wenn die Fusemzellen und die zu immortalisierenden Zellen miteinander ko-kultiviert werden und übertragen durch die Infektion die Immortalisationsgene. Bei der zweiten Methode werden die Fusemzellen mit den zu immortalisierenden Zellen fusioniert, so daß Hybridome entstehen, die sowohl die Merkmale der Fusemzelle als auch der zu immortalisierenden Zelle tragen.

In einer Ausgestaltungsform der Erfindung werden die Hybridomzellen durch die Fusion einer durch EBV konditional immortalisierten B-Zelle mit einer Leukämiezelle gewonnen. Derartige Hybridomzellen können zur Etablierung von T-Zellen eingesetzt werden, die nicht fusionierte Leukämiezellen erkennen können. Das Verfahren, um gegen Tumorzellen, bevorzugt Leukämiezellen, gerichtete T-Zellen herzustellen, kann wie folgt dargestellt werden:
(a) Bereitstellen einer Fusemzelle und einer mit Hilfe der Fusemzelle zu immortalisierenden Tumorzelle;
(b) Übertragung des Merkmals der Immortalisation von Fusemzellen auf die mit Hilfe der Fusemzellen zu immortalisierenden Tumorzellen;
(c) Selektion auf immortalisierte Tumorzellen;
(d) Gewinnung der immortalisierten Tumorzellen, Aufhebung der konditionalen Immortalisation und Ko-Kultivierung mit T-Zellen enthaltenden, peripheren, mononukleären Zellen des Patienten, von welchem die Tumorzelle gewonnen wurde oder eines gesunden Spenders;
(e) Selektion von T-Zellen, die die im Schritt (a) eingesetzten Tumorzellen erkennen können.

Die selektierten T-Zellen können die im Schritt (a) eingesetzten Tumorzellen selektiv erkennen. Selektiv bedeutet hierbei, daß die T-Zellen die im Schritt (a) eingesetzten Tumorzellen, nicht aber die im Schrift (b) Fusemzellen und insbesondere nicht nur die in Schritt (d) eingesetzten immortalisierten Tumorzellen erkennen können.

Falls die gewonnenen T-Zellen für therapeutische Zwecke eingesetzt werden sollen, wird es im allgemeinen wünschenswert sein, sicherzustellen, daß die T-Zellen nur die Tumorzellen und nicht generell alle Zellen des Patienten erkennen können. Auf diese Weise wird die Induktion lebensbedrohender GvH-Zustände vermieden.

In einer weiteren Ausgestaltungsform der Erfindung können durch die Fusion der Fusemzellen mit Lymphozytenzellen Hybridome generiert werden, die beispielsweise einen Antikörper produzieren oder einen T-Zell-Rezeptor mit einer gewünschten Spezifität. Ein derartiges Verfahren kann beispielsweise wie folgt ausgestaltet werden:
(a) Bereitstellen einer Fusemzelle und einer Lymphozytenzelle;
(b) Fusion der Fusemzelle mit der Lymphozytenzelle;
(c) Selektion auf konditional immortalisierte Hybridomzellen und wahlweise Klonierung einzelner Hybridomzellen;
(d) Expansion der Hybridomzellen; und
(e) Aufheben der konditionalen Immortalisierung.

Nachfolgend wird die Erfindung anhand von Ausführungsbeispielen und Abbildungen näher beschrieben. Bei den Ausführungsbeispielen handelt es sich um Ausführungsformen der Erfindung, wobei die Erfindung nicht auf diese speziellen Beispiele beschränkt ist. Im Rahmen der ursprünglichen Offenbarung und der Patentansprüche können diese Beispiele abgeändert werden.

Die Abbildungen zeigen:
Abbildung 1: Einfluß von HAT und 8AG auf EREB2-5-Zellen;
Abbildung 2: Einfluß von Östrogen, HAT und 8AG auf ZAGREB-ZELLEN;
Abbildung 3: Antigene beider Fusionspartner exprimierende Hybridomzellen.

Das im folgenden dargestellte Verfahren bietet eine elegante Methode, um die aus dem Stand der Technik bekannten Probleme zu lösen. Nachfolgend wir die Erfindung dargestellt am Beispiel der Entwicklung eines Prototyps einer universell einsetzbaren Helferzelle zur Immortalisation humaner Zellen (wir nennen diesen Zelltyp ein *Fusem*, da die Fusion dieser Zellen mit geeigneten, zu immortalisierenden Zellen eine bevorzugte Ausgestaltung der Erfindung betrifft.). Weiterhin besteht die Möglichkeit, diese Zellen mit den zu immortalisierenden Zellen zusammen zu kultivieren und durch diese Kokultur einen Transfer der Immortalisation auf die letzteren Zellen zu bewirken). Wesentliche Kennzeichen der *Fuseme* sind einerseits die Konditionalität der Immortalisation und andererseits das Einbringen von zwei Selektionsmarkern in die konditional immortalisierten Zellen. Einer dieser Marker ist geeignet, das Wachstum der *Fuseme* und der Fusionsprodukte aus *Fusem* und zu immortalisierender Zelle in einem Medium zu ermöglichen, welches eine an sonsten toxische Wirkung auf Zellen hat. Der zweite Selektionsmarker ermöglicht die Abtötung nicht fusionierter *Fuseme.* Durch die beiden Selektionsmarker ist eine schnelle und effiziente Trennung von fusionierten und unfusionierten Zellen möglich. Andererseits gestattet die Konditionalität der Immortalisation, daß die fusionierten Zellen nach Abschaltung der Immortalisationsfunktion unabhängig von den immortalisationsbedingten Eigenschaften der Zellen für experimentelle oder therapeutische Anwendungen verwendet werden können.

Für den ersten Selektionsmarker stehen zahlreiche Antibiotikaresistenzen zur Verfügung, z. B. die Geneticin- oder Hygromycinresistenz. Für den zweiten Marker, welcher eine Abtötung der unfusionierten *Fuseme* ermöglicht, bietet sich insbesondere die Sensitivität der Zellen für einen Zusatz von Hypoxanthin, Aminopterin und Thymidin (HAT) zum Kulturmedium an.

Um die entsprechenden Selektionsmarker in die *Fuseme* einzuführen, stehen verschiedene Verfahren zur Verfügung. Unter Einführen im Sinne dieser Anmeldung verstehen wir nicht nur das Einbringen eines entsprechenden Genes in die Zellen mit gentechnischen Methoden, sondern auch die Ausnutzung natürlicher Mutationen, welche Resistenz bzw. Sensitivität gegenüber einem Wirkstoff vermitteln. So lassen sich Zellen in Gegenwart von 8-Azaguanin oder Bromdesoxyuridin kultivieren und dadurch solche Zellen herausselektionieren, welche durch spontane Mutationen einen Defekt in einem bestimmten Stoffwechselweg haben, welcher zur Synthese von DNA-Bausteinen führt. Solchermaßen behandelte Zellen sind anschließend nicht mehr in der Lage, in einem Medium zu wachsen, welches einen alternativen Weg der DNA-Baustein-Sysnthese ebenfalls blockiert. Normale Zellen besitzen jedoch die Fähigkeit, nach Blockade dieses Weges zu wachsen und transferieren diese Eigenschaft auch auf die Fusionsprodukte zwischen diesen Zellen und den *Fusemen*. Ähnliche Wege stehen auch für die Einführung einer spezifischen Resistenz, etwa gegen Oubain, zu Verfügung.

Für die konditionale Immortalisation stehen ebenfalls mehrere Wege offen. Neben Viren, etwa dem EBV, lassen sich auch Onkogene, bzw. Kombinationen mehrerer Onkogene, zur Immortalisation verwenden [MacDonald (1994)]. Gestaltet man die Expression eines solchen Onkogens regulierbar, wie dieses heute problemlos möglich ist [z. B. Wyllie et al. (1993)] , gelangt man auch hier zu konditional immortalisierten *Fusemen*.

Anwendungsgebiete für *Fuseme* sind zahlreich vorhanden. Wie bereits erwähnt, lassen sich durch Einsatz dieser Zellen humane monoklonale Antikörper produzieren, wie sie für zahlreiche Einsatzbereiche, insbesondere in der Klinik, wünschenswert sind. Ein besonders vielversprechendes Anwendungsgebiet liegt in der Stimulation von Immunantworten *in vitro* und *in vivo* gegen Zellen, welche selber nur schlechte immunstimulatorische Eigenschaften haben. Hierbei denken wir vor allem an die Krebstherapie.

Zahlreiche Tumorzellen entgehen der Immunüberwachung dadurch, daß sie kostimulatorische Signale, welche für eine effiziente T-Zell-Stimulation notwendig sind, nicht exprimieren. Andererseits ist die Existenz von tumorspezifischen Antigenen für zahlreiche Tumore nachgewiesen, und ein wesentliches Anliegen der Immuntherapie bei Krebs ist primär die Erhöhung der Immunogenität der Tumorzellen bzw. der Tumorantigene. Zwar sind bereits zahlreiche Tumorantigene kloniert worden, aber für zahlreiche Tumorarten trifft dieses nicht zu und es ist nicht vorhersagbar, daß ein bestimmter Tumor eines bestimmten Patienten auch in jedem Fall das entsprechende Antigen exprimieren muß. Daher ist eine spezifische Therapie im Einzelfall schwierig.

Gerade hier liegt der entscheidende Vorteil der *Fusem*-Technologie. Durch die Fusion und Immortalisation der Tumorzelle werden gleichzeitig Eigenschaften der *Fusem*-Zelle auf die Hybridomzelle übertragen. Im Falle des Einsatzes konditional EBV-transformierter LCL als *Fusem* werden so auch die kostimulatorischen Signale, welche auf der LCL zweifelsfrei vorhanden sind, auf das Hybridom übertragen. Die Tumorantigene werden anderseits von der Tumorzelle auf das Hybridom übertragen. Somit entfällt die Notwendigkeit, zu wissen, welches Tumorantigen in der Tumorzelle überhaupt exprimiert wird. Da die Hybridomzelle konditional immortalisiert ist, lassen sich Antigene, welche immortalisationsspezifisch sind, ausschalten, und hierdurch läßt sich die Immunantwort auf die eigentlichen Tumorantigene fokussieren. Zusätzlich lassen sich die Spezifitäten der etablierten T-Zellen im Anschluß zusätzlich bestimmen und somit weitere Tumorantigene definieren.

### Methode:

### 1.)Etablierung einer konditional immortalisierten universellen Immortalisations-Helferzelle (Fusem).

Geeignete Zellen, z. B. humane Lymphozytenzellen, humane B-Zellen oder T-Zellen, werden mit Hilfe eines geeigneten Verfahrens konditional immortalisiert. Hierzu eignet sich z. B. im Falle von B-Zellen ein EBV, z.B. der EBV-Stamm P3HR1, welcher eine Deletion in dem zur Immortalisation notwendigen Gen EBNA2 trägt. Dieser Virusstamm hat damit seine B-Zell-transformierenden Eigenschaften verloren. Der Defekt läßt sich jedoch komplementieren, wenn das fehlende EBNA2 auf einem anderen Plasmid in dieselbe Zelle gebracht wird. B-Zellen, welche das P3HR1-Virus und zusätzlich ein funktionelles EBNA2 auf einem geeigneten Expressionsvektor enthalten, beginnen unbegrenzt zu proliferieren. Wird anstelle von Wildtyp-EBNA2 ein Fusionsprotein aus EBNA2 und der Hormonbindungs-Domäne des Östrogen-Rezeptors verwendet, gelangt man zwanglos zu konditional immortalisierten Zellen [Kempkes et al. (1995)]. Diese Zellen können dann nur in Anwesenheit von Östrogen im Kulturmedium wachsen und stellen ihr Wachstum nach Östrogenentzug ein. Es ist möglich, auf einem weiteren oder demselben Plasmid, welches die Expression des ER/EBNA2-Fusionsproteins ermöglicht ein Resistenzgen für einen geeigneten Wirkstoff, z. B. Geneticin, unterzubringen. Dadurch werden die konditional immortalisierten Zellen zugleich mit einem positiven Selektionsmarker für die spätere Selektion der Hybridome ausgestattet.

Durch Kultur der so etablierten konditional immortalisierten Zellen in Anwesenheit von 8-Azaguanin oder Bromdesoxyuridin selektioniert man auf Zellen, welche resistent gegenüber diesem Wirkstoff sind. Diese Zellen werden dadurch gleichzeitig sensitiv für eine Mischung aus Hypoxanthin, Aminopterin und Thymidin (HAT). Hierdurch werden die Zellen mit einem zweiten Selektionsmarker, in diesem Falle zur Negativselektion, ausgestattet. Das Produkt stellt das gewünschte *Fusem* dar.

### 2.)Immortalisationstransfer auf die zu immortalisierenden Zellen und Selektion der immortalisierten Zellen.

### a) Transfer durch Zellfusion.

Bei dieser Methode erfolgt die Immortalisation der gewünschten Zelle durch Fusion mit dem *Fusem.* Diese Fusion kann beispielsweise durch Polyethylenglycol ausgelöst werden. Da das *Fusem* resistent gegenüber z. B. Geneticin und 8-Azaguanin ist, lassen sich die entstandenen Hybridome alsbald von unfusionierten *Fusemen* und unfusionierten anderen Zellen durch Kultur in HAT und Geneticin selektionieren.

### b) Transfer durch Kokultur.

Wenn die zu immortalisierende Zelle und das *Fusem* demselben primären Zelltyp entsprechen (z.B. beides Abkömmlinge von B-Zellen) und die Immortalistaion des *Fusems* mit Hilfe transformierender Viren durchgeführt wurde, welche aus dem *Fusem* als infektiöse Partikel freigesetzt werden können, so ist der Immortalisations-Transfer dadurch möglich, daß das *Fusem* mit der zu immortalisierenden Zelle kokultiviert wird. Da das *Fusem* resistent gegenüber 8-Azaguanin ist, lassen sich die entstandenen immortalisierten Zellen von restlichen Helferzellen durch Kultur in Anwesenheit von HAT trennen. Sollte das Problem bestehen, daß andere, ungewünschte Zellen (bei Immortalisation von Zellen aus dem Blut beispielsweise kontaminierende T-Zellen oder endogene EBV-transfomierte Zellen) das Wachstum der Zellen ungünstig beeinflussen, so kann durch Verwendung des zweiten, positiven Selektionsmarkers, also z. B. Geniticin, ein Wachstum dieser Zellen wirkungsvoll verhindert werden.

Da die *Fuseme* mit einem Negativ-Selektionsmarker ausgestattet sind (beispielsweise HAT-Sensitivität), besteht die Möglichkeit, die Immortalisation einer Zielzelle in einem Zwei-Schrift-Verfahren durchzuführen, wenn dieses gewünscht ist. Voraussetzung hierzu ist allerdings, daß die konditionale Immortalisation des *Fusems* mit Hilfe eines geeigneten Vektors durchgeführt wurde, welcher aus dem *Fusem* freigesetzt werden kann und in der Lage ist, eine neue Zelle des gleichen Zelltyps erneut konditional zu immortalisieren. Für diese Methode benötigt man lediglich einen Prototyp eines Fusems, dessen Etablierung durch uns im folgenden beschrieben ist.

### Etablierung eines Prototyp-Fusems:

Es wird die Etablierung eines *Fusems* beschrieben, welches erfindungsgemäß generiert wurde und zur Zeit die erste und einzige Zell-Linie dieser Art darstellt. Dieses *Fusem* wurde ZAGREB genannt. Zur Etablierung der ZAGREB-*Fusem*-Linie wurde von einer Zell-Linie (EREB2-5) ausgegangen, welche bereits beschrieben wurde [Kempkes et al. (1995)]. Diese Zellen entstanden durch gleichzeitige Infektion von Nabelschnurblut-Zellen mit dem P3HR1-EBV-Virus und einem Expressionsplasmid, welches die Expression eines ER/EBNA2-Fusionsproteins erlaubt. Diese Zellen wachsen nur in Anwesenheit von Östrogen im Kulturmedium. Nach Östrogenentzug werden zahlreiche EBV-Antigene nicht mehr exprimiert, und die Expression des endogenen Immunglobulins wird deutlich gesteigert. Damit erfüllt diese Zelle gleich mehrere positive Eigenschaften, welche sie für die folgende Etablierung eines *Fusems* geeignet erscheinen ließen. Da diese Zellen zugleich resistent gegenüber Geneticin sind, erübrigte sich das Einbringen eines positiven Selektionsmarkers in die Zellen.

Hierzu wurden die Zellen (4x10⁷ in 40 ml RPMI1640 + 10% FKS + Pen/Strep, 50U (µg)/ml + Amphotericin B 750 ng/ml) in Anwesenheit von Östrogen (1µM) und 8-Azaguanin (8AG, 6,6 x 10⁻³ M) kultiviert. Unter diesen Bedingungen starben die meisten Zellen innerhalb der ersten 4 Wochen. Zu diesem Zeitpunkt wurden die überlebenden Zellen abzentrifugiert (1000 rpm, 10 min) und in 20 ml frischem Medium mit denselben Zusätzen resuspendiert. Durch den Mediumwechsel und das frische Östrogen begannen die Zellen massiv zu proliferieren. Unseren überraschenden Beobachtungen nach ist es wesentlich leichter, konditional immortalisierte LCL 8AG-resistent zu machen, als klassische LCL.

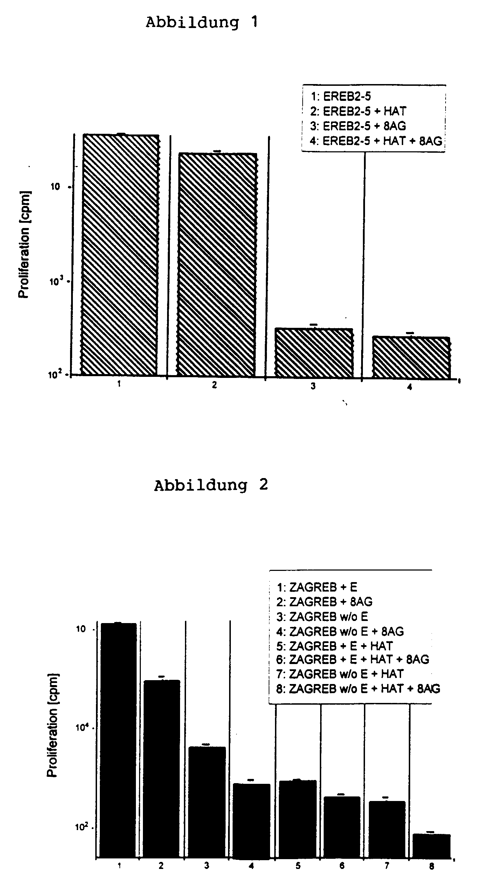

Die etablierte 8AG-resistente Zell-Linie (ZAGREB) wurde auf ihre HAT-Sensitivität getestet. Wie in Abb. 1 gezeigt, ist die Ausgangszell-Linie EREB2-5 resistent gegenüber HAT und sensitiv gegenüber 8AG. Im Gegensatz hierzu sind ZAGREB-Zellen 8AG-resistent und HAT-sensitiv (Abb 2.). Wie ebenfalls in Abb. 2 gezeigt, bleibt jedoch die Konditionalität der Immortalisation erhalten. In Abwesenheit von Östrogen stellen die Zellen ihr Wachstum ein. Somit stellen ZAGREB-Zellen einen Prototyp für ein *Fusem* dar.

Die Fusion dieser Zellen mittels PEG ist problemlos möglich. Als Fusionspartner wurden PBMC eines gesunden Spenders, eine Burkitt-Lymphom-Zell-Linie sowie ein humanes Thymom getestet.

Die Immortalisation von Zellen durch Kokultur wurde mittels PBMC eines gesunden Spenders sowie einer humanen Zell-Linie einer Chronisch Lymphatischen Leukämie ausgetestet.

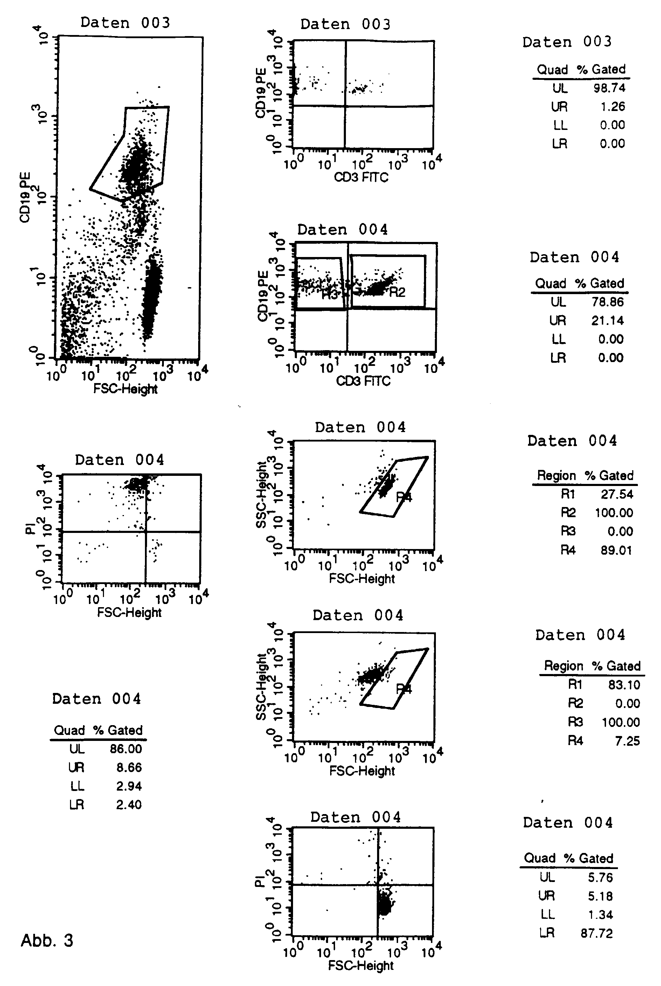

Beispielhaft ist in Fig. 3 das Ergebnis einer Fusion zwischen ZAGREB-Zellen und der humanen Thymomzell-Linie Jurkat gezeigt. Diese Fusion erfolgte nach einem Verfahren, welches zur Steigerung der Fusionseffizienz die ConA-vermittelte Adhäsion der Zellen an die Zellkulturschale verwendet (Graessmann et al. (1980)]. Unmittelbar nach der Fusion wurde die Selektion mit HAT (5 x 10⁻³ M Hypoxantin, 2 x 10⁻⁵ M Aminopterin, 8 x 10 ⁻⁴ M Thymidin). Nach weitem 2 Tagen wurden die Zellen mit Geneticin (250 µg / ml) behandelt, um das Wachstum der unfusionierten Jurkat-Zellen zu unterbinden. Wie deutlich zu sehen ist, sind eine Woche nach der Fusion Zellen nachweisbar, welche positiv sowohl für CD19 (ein Marker der ZAGREB-Zellen) als auch für CD3 (ein Marker der Jurkat-Zellen) sind. Ebenfalls deutlich ist, daß der größte Teil der CD19-positiven Zellen eine deutlich geringere Vorwärtsstreuung (FSC) zeigen, als die CD19-negativen Zellen. Dies ist ein Zeichen für die beginnende Apoptose dieser Zellen, ausgelöst durch die HAT-Selektion, eine Interpretation, die durch die ebenfalls gezeigte Propidium-Jodid-Färbung unterstützt wird [Darzynkiewicz et al. (1995)]. Somit eignet sich das *Fusem*, um Hybridome mit anderen Zellen, in diesem Falle einem Lymphom, zu bilden. Es kann davon ausgegangen werden, daß die Hybridomzellen Eigenschaften beider Fusionspartner haben (siehe CD19 und CD3). Da CD3 mit dem idiotypischen (und damit tumorspezifischen) T-Zellrezeptor assoziiert auf der Zellmembran erscheint, zeigt dieses Beispiel zugleich, daß die Hybridome ein tumorspezifisches Antigen weiterhin exprimieren können.

Dieses *Fusem* eignet sich als Helferzelle für die Generierung weiterer *Fuseme* mit gewünschten Eigenschaften, um diese mit den finalen Zielzellen zu fusionieren. Beispielsweise können von einem Patienten *Fuseme* erzeugt werden, indem in einem ersten Schrift die PBMC des Patienten zusammen mit ZAGREB-Zellen kokultiviert werden. Durch Zusatz von HAT zum Kulturmedium wird das Wachstum der ZAGREB-Zellen verhindert. Die aus den ZAGREB-Zellen freigesetzten EBV-Partikel können B-Zellen des Patienten infizieren und konditional transformieren. Sobald Wachstum der Zellen beobachtet wird, kann mittels Geneticinzugabe zum Kulturmedium das Wachstum aller Zellen unterdrückt werden, welche proliferieren, ohne das ER/EBNA2-Konstrukt zu tragen (z. B. alloreaktive und EBV-spezifische T-Zellen oder endogen mit EBV infizierte B-Zellen des Patienten), da auf diesem Konstrukt die Geneticin-Resistenz kodiert wird. Durch Kultur der Zellen in 8AG gelangt man zu Zellen, welche sich wie ZAGREB-Zellen verhalten, aber die genetischen Marker (z. B. MHC) des Patienten tragen. Diese Zellen können daraufhin mit den Tumorzellen des Patienten fusioniert werden und als Vakzine eingesetzt werden. Einzige Voraussetzung für das Gelingen dieses Ansatzes ist, daß genügend Zeit für den zweifachen Immortalisationstransfer zur Verfügung steht (es werden bei optimalem Verlauf ca. 8 Wochen benötigt, 4 Wochen zur Etablierung des *Fusems* und 4 Wochen um dieses *Fusem* HAT-sensitiv zu machen).

### Anwendungsbeispiel:

Leukämiezellen eines Patienten mit Akuter Lymphatischer Leukämie (ALL) werden aus dem peripheren Blut gewonnen. Ein Teil der Zellen wird in flüssigem Stickstoff asserviert. Ein zweiter Teil der Zellen wird mit einem *Fusem* fusioniert. Als *Fusem* werden in diesem Falle z. B. ZAGREB-Zellen oder andere konditional EBV-immortalisierte B-Zellen mit den Eigenschaften der ZAGREB-Zell-Linie verwendet. Ein Immortalisationstransfer mittels Kokultur ist in diesem Falle unseres heutigen Wissens nach nicht möglich, da ALL-Zellen keinen Rezeptor für EBV tragen. Anschließend werden die gewünschten Hybridome mittels Kultur in Gegenwart von HAT und Geneticin selektioniert. Unterdessen hat der Patient eine Knochenmarktransplantation (KMT) von einem geeigneten Spender erhalten. Periphere Mononukleäre Zellen (PBMC) des KM-Spenders werden zusammen mit den Hybridomzellen nach konditionaler Abschaltung der Immortalisation kultiviert Hierdurch werden die in den PBMC enthaltenen T-Zellen sowohl gegenüber Antigenen, welche von dem *Fusem* stammen (jedoch nicht gegenüber Antigenen, deren Expression abhängig von der Immortalisation des *Fusems* sind) als auch gegenüber Antigenen, welche in den Leukämiezellen exprimiert werden, sensibilisiert. Anschließend wird mit geeigneten Methoden (⁵¹Cr-Freisetzungstest, Proliferations-Test, etc.) getestet, ob die durch dieses Verfahren generierten T-Zellen in der Lage sind, die unfusionierten Leukämiezellen (welche in der Zwischenzeit in flüssigem Stickstoff asserviert waren) zu erkennen. Im positiven Fall werden die T-Zellen durch repetitive Restimulationen expandiert und auf ihre mögliche Graft-versus-Host (GvH)-Aktivität getestet. Im Falle fehlender GvH-Aktivität werden die generierten T-Zell-Linien in flüssigem Stickstoff asserviert. Sobald der Patient ein Rezidiv hat, werden die generierten T-Zellen des KM-Spenders dem Patienten adoptiv transferiert, um den Heilungsprozeß zu initiieren. Die etablierte *Fusem*/Leukämie-Hybridom-Zell-Linie kann benutzt werden, um a) biochemische, zellbiologische, molekular- und cytogenetische Untersuchungen durchzuführen, b) Tumorantigene zu charakterisieren und c) als allogene Vakzine für Patienten mit ähnlichen MHC-Allelen und derselben Grunderkrankung wie der Patient zu dienen.

Die Erfindung umfaßt die nachfolgenden bevorzugten Ausgestaltungen:
- Ein Verfahren zur Herstellung einer konditional immortalisierten Immortalisations-Helferzelle (Fusem) mit zumindest den nachfolgenden Schritten:
   (a) Einführen von Immortalisierungs-Genen in Säugerzellen in solcher Weise, daß zumindest die Expression und/oder Funktion zumindest eines dieser Gene regulierbar ist, um konditional immortalisierte Säugerzellen zu erhalten;
   (b) Einführen von mindestens zwei Selektionsmarkern für eine Positiv- und eine Negativselektion, um eine Selektion zwischen den Fusemzellen, den mit Hilfe der Fusemzellen zu immortalisierenden Zellen und den mit Hilfe der Fusemzellen immortalisierten Zellen zu ermöglichen;
   (c) Selektion auf solche Zellen (Fuseme), die die im Verfahrensschritt (b) eingeführten Selektionsmarker tragen und konditional immortalisiert sind.
- Als Säugerzellen werden bevorzugt menschliche Zellen oder Zellen aus Nagern eingesetzt, bevorzugt Lymphozyten-Zellen oder Fibroblastenzellen eingesetzt.
- Als Immortalisierungsgene werden bevorzugt Gene des Epstein-Barr-Virus (EBV), Gene von Adenoviren, HTLV-1 oder Onkogene eingesetzt.
- Als Vektoren zum Einführen der Immortalisierungsgene werden bevorzugt von EBV, von Adenoviren, von Retroviren, von Foamyviren, Poxviren oder von SV40 abgeleitete Vektoren verwendet.
- Als von EBV abgeleitete Vektoren werden bevorzugt mini-EBV-Vektoren verwendet werden und/oder als von Poxviren abgeleitete Vektoren Vacciniaviren verwendet.
- Als das regulierbare Immortalisierungsgen des EBV wird bevorzugt das EBNA2-Gen, das LMP1-Gen oder das EBNA3a-Gen verwendet.
- Die regulierbaren Immortalisierungsgene sind bevorzugt durch Hormone oder Antibiotika regulierbar.
- Als Hormon wird bevorzugt ein Östrogen oder ein Androgen verwendet.
- Als Selektionsmarker für eine Positivselektion werden bevorzugt die Zellen gegenüber einem Agens resistent und für eine Negativselektion sensitiv gegenüber einem Agens gemacht.
- Die Zellen werden bevorzugt gegenüber einem Antibiotikum resistent und für HAT sensitiv gemacht.
- Die Zellen werden bevorzugt resistent gegenüber Geneticin, Hygromycin oder Oubain gemacht.
- Die Schritte (a) und (b) werden bevorzugt gleichzeitig durchgeführt.
- Als regulierbares Immortalisationsgen wird z.B. das EBNA2-Gen von EBV, als Positivselektionsmarker ein Antibiotikumresistenzgen, als Negativselektionsmarker eine Sensitivität gegenüber HAT, als Virus zum Einführen des Immortalisierungsgens EBV und als Säugerzelle eine B-Zelle eingesetzt.

Die Säugerzellen werden z. B. durch Zugabe von 8-Azaguanin oder Bromdesoxyuridin sensitiv gegenüber HAT gemacht.

Fusemzelle, gekennzeichnet zumindest durch die nachfolgenden Merkmale:
(a) Säugerzelle, die konditional immortalisiert ist;
(b) mit zumindest zwei Selektionsmarkern für eine Positiv- und eine Negativselektion, die eine Selektion zwischen Fusemzellen, mit Hilfe der Fusemzellen zu immortalisierenden Zellen und mit Hilfe der Fusemzellen immortalisierten Zellen ermöglichen.

- Verwendung dieser Fusemzelle zur Herstellung einer monoklonale Antikörper produzierenden Zellinie oder zur Herstellung einer T/B-Hybridomzellinie.
- Verfahren zur Immortalisation von Säugerzellen mit den nachfolgenden Schritten:
   (a) Bereitstellen einer Fusemzelle und einer zu immortalisierenden Säugerzelle;
   (b) Übertragung des Merkmals der Immortalisation von den Fusemzellen auf die zu immortalisierenden Zellen;
   (c) Selektion auf Zellen, die das Merkmal der Immortalisation tragen.
- Als zu immortalisierende Säugerzelle wird bevorzugt eine Lymphozyten-Zelle oder eine Fibroblastenzelle eingesetzt.
- Als zu immortalisierende Zelle wird bevorzugt eine Tumorzelle eingesetzt z.B. eine Leukämiezelle.
- Die Übertragung des Merkmals der Immortalisation erfolgt z. B. durch Gentransfer von dem Fusem auf die zu immortalisierende Zelle.
- Der Gentransfer erfolgt z. B. durch aus dem Fusem freigesetzte, die Im mortalisierungsgene enthaltende, transferierende Viren und Infektion der zu immortalisierenden Zellen durch die transferierenden Viren.
- Die Fusemzellen und die zu immortalisierenden Zellen werden bevorzugt kokultiviert.
- Die Übertragung des Merkmals der Immortalisation erfolgt z. B. durch Fusion der Fusemzellen und der zu immortalisierenden Zellen.
- Die Zellfusion wird z. B. durch Polyethylenglycol, Elektrofusion oder Viren eingeleitet.
- Die Selektion auf immortalisierte Zellen erfolgt z. B. durch Kultivierung der Zellen in einem Medium, welches weder das Wachstum der Fuseme noch das Wachstum der zu immortalisierenden Zellen erlaubt.
- Die Selektion der zu immortalisierenden Zellen erfolgt durch Kultur in HAT-Medium.
- Hybridomzelle, bestehend aus dem Fusionsprodukt einer Fusemzelle und einer zu immortalisierenden Säugerzelle, z. B. einer Tumorzelle, z. B. einer Leukämiezelle.
- Hybridomzelle, wobei die Fusemzelle aus einer durch EBV konditional immortalisierten B-Zelle gewonnen wurde.
- Verwendung einer oben genannten Hybridomzelle zur Stimulation von T-Zellen, welche nach der Stimulation nicht fusionierte Tumorzellen erkennen können.
- Verfahren zur Schaffung von gegen Tumorzellen gerichteten T-Zellen mit den nachfolgenden Schritten:
   (a) Bereitstellen einer Fusemzelle und einer zu immortalisierenden Tumorzelle;
   (b) Übertragung des Merkmals der Immortalisation von den Fusemzellen auf die zu immortalisierenden Tumorzellen;
   (c) Selektion auf immortalisierte Tumorzellen;
   (d) Gewinnung der immortalisierten Tumorzellen, Aufhebung der konditionalen Immortalisation und Ko-Kultivierung mit T-Zellen enthaltenden, peripheren, mononukleären Zellen des Patienten, von welchem die Tumorzelle gewonnen wurde oder eines gesunden Spenders;
   (e) Selektion von T-Zellen, die die im Schritt (a) eingesetzten Tumorzellen selektiv erkennen können.
- Als Tumorzellen werden z. B. Leukämiezellen oder Lymphomzellen eingesetzt.
- Verfahren aus einer Fusemzelle und einer Lymphozytenzelle mit den nachfolgenden Schritten:
   (a) Bereitstellen einer Fusemzelle und einer Lymphozytenzelle;
   (b) Fusion der Fusemzelle mit der Lymphozytenzelle;
   (c) Selektion auf konditional immortalisierte Hybridomzellen und wahlweise Klonierung einzelner Hybridomzellen;
   (d) Expansion der Hybridomzellen; und
   (e) Aufheben der konditionalen Immortalisierung.
- Die Lymphozyten sind bevorzugt in den peripheren, mononukleären Zellen eines Spenders enthalten.
- Im Schritt (c) auf konditional immortalisierte Hybridomzellen selektiert wird, die einen Antikörper mit einer bestimmten Spezifität exprimieren oder die einen T-Zell-Rezeptor mit einer bestimmten Spezifität enthalten.

- **Abb. 1.**: **Einfluß von HAT und 8AG auf EREB2-5-Zellen.** EREB2-5-Zellen (2x10⁴ / well) wurden in An- bzw. Abwesenheit von HAT bzw 8AG für 5 Tage in Mikrotiterplatten kultiviert, davon die letzten 24 h in Anwesenheit von ³H-Thymidin. Der Einbau von radioaktivem Thymidin in die DNA wurde mittels Flüssigszintillationsmessung nachgewiesen.

**Abb. 2. Einfluß von Östrogen, HAT und 8AG auf ZAGREB-Zellen.** ZAGREB-Zellen (2x10⁴ / well) wurden in An- bzw. Abwesenheit von Östrogen, HAT bzw 8AG für 5 Tage in Mikrotiterplatten kultiviert, davon die letzten 24 h in Anwesenheit von ³H-Thymidin. Der Einbau von radioaktivem Thymidin in die DNA wurde mittels Flüssigszintillationsmessung nachgewiesen.
**Abb. 3: Hybridom-Zellen exprimieren Antigene beider Fusionspartner.** Jurkat-Zellen wurden mit Zagreb-Zellen fusioniert und nach einer Woche HAT/Geneticin-Selektion mittels FACS analysiert. Hierzu wurden die Zellen mit Medium (DATA.003) oder einem Antikörper mit Spezifität für CD3 (DATA.004) inkubiert, gewaschen und gebundener Antikörper mittels eines FITC-markiertem Sekundärantikörpers nachgewiesen. Anschließend wurden die Zellen mit einem PE-markiertem Antikörper mit Spezifität für CD19 inkubiert und gewaschen. Zellen, welche positiv für CD19 waren wurden elektronisch eingegatet (Oben links) und der Prozentsatz der CD19-positiven Zellen, welche gleichzeitig CD3-positiv waren wurde bestimmt. Deutlich zu sehen ist die doppelt positive Population von Zellen, welche den fusionierten Zellen entsprechen. Ebenfalls deutlich zu sehen ist (untere Bildhälfte), daß die doppelt positiven Zellen eine höhere Vorwärtsstreuung des Lichtes (FSC) und nach Färbung mit Propidium-Iodid (PI) eine geringere Fluoreszenz zeigen, als die einfach CD19-positiven Zellen.Diese beiden Parameter zeigen, daß die einfach CD19-positiven Zellen apoptotisch werden, was anläßlich der HAT-Selektion auch so zu erwarten ist.
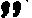 **QUAD"**: ist die Abkürzung für Quadrant (UL=oben links; LR=unten rechts etc);
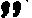 **% Gated"** ist die Prozentzahl der Zellen, welche sich jeweils in einem der entsprechenden vier Quadranten befinden;
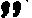 **FSC-High"** meint die Vorwärtsstreuung des Lichts, ein Maß für die Zellgröße;
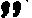 **SSC-High"** Seitwärtsstreuung des Lichts, ein Maß für die Granulierung der Zellen;
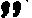 **PI."** Propidium-Iodid
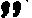 **PE"** Phycoerythrin
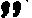 **FITC"** Fluoreszein-Isothiozyanat

### Literatur:

Darzynkiewicz, Z., X. Li, J. Gong, S. Hara & F. Traganos, 1995. Analysis of cell death by flow cytometry. *In*: G. P. Studzinski (Hrsg.), Cell growth and apoptosis (A practical approach). Oxford, IRL Press.
Gordon, J., 1989. Human monoclonal antibodies. *In*: D. Catty (Hrsg.), Antibodies, Bd. 1 (A practical approach). Oxford, IRL Press.
Graessmann, A., H. Wolf & G. W. Bornkamm, 1980. Expression of Epstein-Barr virus genes in different cell types after microinjection of viral DNA. Proc. Natl. Acad. Sci. USA 77:433.
Hubbard, K. & H. L. Ozer, 1995. Senescence and immortalization of human cells. *In*: G. P. Studzinski (Hrsg.), Cell growth and apoptosis (A practical approach). Oxford, IRL Press.
Kempkes,B., D. Spitkovsky, P. Jansen-Dürr, J. W. Ellwart, E. Kremmer, H.-J. Delecluse, C. Rottenberger, G. W. Bornkamm & W. Hammerschmidt, 1995. B-cell proliferation and induction of early G1-regulating proteins by Epstein-Barr Virus mutants conditional for EBNA2. EMBO J. 14:88.
Köhler, G & C. Milstein, 1975. Continuous culture of fused cells secreting antibody of predifined specificity. Nature 256:495.
Lange, B. J., 1989. Growth of human leukaemia cells *in vitro. In*: R. Baserga (Hrsg.), Cell growth and division (A practical approach). Oxford, IRL Press.
MacDonald, C, 1994. Immortalization of hematopoietic cells. In: Freshney, R. I., I. B. Pragnell & M. G. Freshney (Hrsg.), Culture of hematopoietic cells. New York, Wiley-Liss.
Peters, J. H., H. Baumgarten & M. Schulze, 1988. Monoklonale Antikörper. Berlin, Springer.
Visonneau, S., A. Cesano & D. Santoli, 1995. Growth and activation of human leukaemic cells *in vitro* and their growth in the SCID mouse model. *In*: G. P. Studzinski (Hrsg.), Cell growth and apoptosis (A practical approach). Oxford, IRL Press.
Walls, E. V. & D. H. Crawford, 1987. Generation of human B lymphoblastoid cell lines using Epstein-Barr virus. *In*: G. G. B. Klaus (Hrsg.), Lymphocytes (A practical approach). Oxford, IRL Press.
Wyllie, F. S., J. A. Bond, T. Dawson, D. White, R. Davies & D. Wynford-Thomas, 1993. A phenotypically and karyotypically stable human thyroid endothelial line conditionally immortalized by SV40T. *In*: N. Lemoine & A. Epenetos (Hrsg.), Mutant oncogenes. London, Chapman & Hall.

## Patentansprüche

1. Verfahren zur Herstellung einer konditional immortalisierten Immortalisations-Helferzelle (Fusem) mit zumindest den nachfolgenden Schritten:
(a) Einführen von Immortalisierungs-Genen in Säugerzellen in solcher Weise, daß zumindest die Expression und/oder Funktion zumindest eines dieser Gene regulierbar ist, um konditional immortalisierte Säugerzellen zu erhalten;
(b) Einführen von mindestens zwei Selektionsmarkern für eine Positiv- und eine Negativselektion, um eine Selektion zwischen den Fusemzellen, den mit Hilfe der Fusemzellen zu immortalisierenden Zellen und den mit Hilfe der Fusemzellen immortalisierten Zellen zu ermöglichen;
(c) Selektion auf solche Zellen (Fuseme), die die im Verfahrensschritt (b) eingeführten Selektionsmarker tragen und konditional immortalisiert sind.

2. Verfahren nach Anspruch 1,
dadurch gekennzeichnet, daß
als Immortalisierungsgene Gene des Epstein-Barr-Virus (EBV), Gene von Adenoviren, HTLV-1 oder Onkogene eingesetzt werden.

3. Verfahren nach Anspruch 1 oder 2,
dadurch gekennzeichnet, daß
als das regulierbare Immortalisierungsgen des EBV das EBNA2-Gen, das LMP1-Gen oder das EBNA3a-Gen verwendet wird.

4. Verfahren nach einem oder mehreren der vorhergehenden Ansprüche,
dadurch gekennzeichnet, daß
die regulierbaren Immortalisierungsgene durch Hormone oder Antibiotika regulierbar sind.

5. Verfahren nach einem oder mehreren der vorhergehenden Ansprüche,
dadurch gekennzeichnet, daß
als Selektionsmarker für eine Positivselektion die Zellen gegenüber einem Agens resistent gemacht werden und für eine Negativselektion sensitiv gegenüber einem Agens gemacht werden.

6. Verfahren nach Anspruch 5,
dadurch gekennzeichnet, daß
die Zellen gegenüber einem Antibiotikum resistent gemacht werden und für HAT sensitiv gemacht werden.

7. Verfahren nach einem oder mehreren der vorhergehenden Ansprüche,
dadurch gekennzeichnet, daß
die Schritte (a) und (b) gleichzeitig durchgeführt werden.

8. Fusemzelle, gekennzeichnet zumindest durch die nachfolgenden Merkmale:
(a) Säugerzelle, die konditional immortalisiert ist;
(b) mit zumindest zwei Selektionsmarkern für eine Positiv- und eine Negativselektion, die eine Selektion zwischen Fusemzellen, mit Hilfe der Fusemzellen zu immortalisierenden Zellen und mit Hilfe der Fusemzellen immortalisierten Zellen ermöglichen.

9. Verfahren zur Immortalisation von Säugerzellen mit den nachfolgenden Schritten:
(a) Bereitstellen einer Fusemzelle und einer zu immortalisierenden Säugerzelle;
(b) Übertragung des Merkmals der Immortalisation von den Fusemzellen auf die zu immortalisierenden Zellen;
(c) Selektion auf Zellen, die das Merkmal der Immortalisation tragen.

10. Verfahren nach Anspruch 9,
dadurch gekennzeichnet, daß
die Übertragung des Merkmals der Immortalisation durch Gentransfer von dem Fusem auf die zu immortalisierende Zelle erfolgt.

11. Verfahren nach Anspruch 10,
dadurch gekennzeichnet, daß
der Gentransfer durch aus dem Fusem freigesetzte, die Immortalisierungsgene enthaltende, transferierende Viren und Infektion der zu immortalisierenden Zellen durch die transferierenden Viren erfolgt.

12. Verfahren nach Anspruch 11,
dadurch gekennzeichnet, daß
die Fusemzellen und die zu immortalisierenden Zellen kokultiviert werden.

13. Verfahren nach Anspruch 9,
dadurch gekennzeichnet, daß
die Übertragung des Merkmals der Immortalisation durch Fusion der Fusemzellen und der zu immortalisierenden Zellen erfolgt.

14. Verfahren nach einem oder mehreren der vorhergehenden Ansprüche 9 - 13,
dadurch gekennzeichnet, daß
die Selektion auf immortalisierte Zellen durch Kultivierung der Zellen in einem Medium erfolgt, welches weder das Wachstum der Fuseme noch das Wachstum der zu immortalisierenden Zellen erlaubt.

15. Hybridomzelle, bestehend aus dem Fusionsprodukt einer Fusemzelle und einer zu immortalisierenden Säugerzelle.

16. Verfahren zur Schaffung von gegen Tumorzellen gerichteten T-Zellen mit den nachfolgenden Schritten:
(a) Bereitstellen einer Fusemzelle und einer zu immortalisierenden Tumorzelle;
(b) Übertragung des Merkmals der Immortalisation von den Fusemzellen auf die zu immortalisierenden Tumorzellen;
(c) Selektion auf immortalisierte Tumorzellen;
(d) Gewinnung der immortalisierten Tumorzellen, Aufhebung der konditionalen Immortalisation und Ko-Kultivierung mit T-Zellen enthaltenden, peripheren, mononukleären Zellen des Patienten, von welchem die Tumorzelle gewonnen wurde oder eines gesunden Spenders;
(e) Selektion von T-Zellen, die die im Schritt (a) eingesetzten Tumorzellen erkennen können.

17. Verfahren zur Herstellung von Hybridomzellen aus einer Fusemzelle und einer Lymphozytenzelle mit den nachfolgenden Schritten:
(a) Bereitstellen einer Fusemzelle und einer Lymphozytenzelle;
(b) Fusion der Fusemzelle mit der Lymphozytenzelle;
(c) Selektion auf konditional immortalisierte Hybridomzellen und wahlweise Klonierung einzelner Hybridomzellen;
(d) Expansion der Hybridomzellen; und
(e) Aufheben der konditionalen Immortalisierung.

18. Verfahren nach Anspruch 17,
dadurch gekennzeichnet, daß
die Lymphozyten in den peripheren, mononukleären Zellen eines Spenders enthalten sind.

19. Verfahren nach Anspruch 17 oder 18,
dadurch gekennzeichnet, daß
im Schritt (c) auf konditional immortalisierte Hybridomzellen selektiert wird, die einen Antikörper mit einer bestimmten Spezifität exprimieren oder die einen T-Zell-Rezeptor mit einer bestimmten Spezifität enthalten.
